# EUROPEAN PATENT APPLICATION

(11) **EP 3 831 361 A1**
(43) Date of publication of application: **09.06.2021**
(21) Application number: 19844716.1
(22) Date of filing: 30.07.2019
(51) Int. Cl.: A61K 8/894, A61K 8/06, A61K 8/37, A61K 8/81, A61K 8/891, A61Q 1/00

(54) **OIL-IN-WATER EMULSION COSMETIC**

(30) Priority: 01.08.2018 JP 2018145176
(71) Applicant: Shiseido Company, Ltd., Chuo-ku Tokyo 104-0061 (JP)
(72) Inventor: MATSUFUJI Yoko, Tokyo 104-0061 (JP); HATA Hideo, Tokyo 104-0061 (JP)
(74) Representative: Ter Meer Steinmeister & Partner
(86) International application number: PCT/JP2019/029914
(87) International publication number: WO 2020/027164

(57) **Abstract**

The present invention pertains to an oil-in-water emulsion cosmetic that comprises: 2-10 mass% of a self-underwater-dispersion-type cross-linked silicone elastomer; a water-soluble macromolecule having emulsification capacity and/or a non-ionic surfactant having an HLB of at least 12.5; and silicone oil having a kinematic viscosity of at most 20 mm2/s at 25°C, wherein the kinematic viscosity of the cosmetic is at most 10,000 mPa·s at 25°C.

## Description

### TECHNICAL FIELD

The present invention relates to an oil-in-water emulsion cosmetic and specifically relates to an oil-in-water emulsion cosmetic which has excellent storage stability and a low viscosity and achieves excellent smoothness/evenness of makeup application as well as having the conventional excellent feeling upon use (reduced stickiness or sliminess) and the uneven-texture correcting effect.

### BACKGROUND ART

Oil-in-water emulsion cosmetics are known as cosmetics which provide excellent feeling upon use (reduced stickiness or sliminess) and an effect of correcting uneven texture, and for example, PTL 1 discloses an oil-in-water emulsion skin cosmetic containing a crosslinked methylpolysiloxane as an oil-in-water emulsion cosmetic having excellent feeling upon use and excellent storage stability.

### CITATION LIST

### PATENT LITERATURE

PTL 1: Japanese Patent No. 5588164

### SUMMARY OF INVENTION

### TECHNICAL PROBLEM

In order to stably disperse or blend an oil-in-water emulsion cosmetic containing the conventional crosslinked silicone elastomer such as crosslinked methylpolysiloxane, however, it has been necessary to blend a certain amount or more of a surfactant having a hydrophilic moiety and a hydrophobic moiety because the crosslinked silicone elastomer itself is not dispersible in water.

Moreover, because there are concerns of precipitation or agglutination with time even when the cosmetic is excellently dispersed, excellent storage stability has been achieved by deliberately increasing the viscosity and thus providing an oil-in-water emulsion cosmetic with a high viscosity.

Considering the above points, the present inventors have thought that cosmetics which have further improved feeling upon use and achieve excellent smoothness/evenness of makeup application as well as having the conventional excellent feeling upon use and the uneven-texture correcting effect would be obtained when an oil-in-water emulsion cosmetic which has a low viscosity but has excellent storage stability could be obtained. That is, an object of the invention is to obtain an oil-in-water emulsion cosmetic which has excellent storage stability and a low viscosity and also achieves excellent smoothness/evenness of makeup application.

### SOLUTION TO PROBLEM

As a result of intensive investigation to achieve the object, the inventors have found that an oil-in-water emulsion cosmetic which has a low viscosity but has excellent storage stability can be obtained when a self-water-dispersible crosslinked silicone elastomer is used and thus have completed the invention.

That is, the invention has the following constitutions.
<1> An oil-in-water emulsion cosmetic containing 2 to 10 mass% of a self-water-dispersible crosslinked silicone elastomer, at least one of a nonionic surfactant having HLB of 12.5 or more and a water-soluble polymer having emulsifying capability and a silicone oil having a kinematic viscosity at 25°C of 20 mm²/s or less, wherein the oil-in-water emulsion cosmetic has a kinematic viscosity at 25°C of 10000 mPa·s or less.
<2> The oil-in-water emulsion cosmetic according to <1>, wherein the self-water-dispersible crosslinked silicone elastomer contains polyethylene glycol as a hydrophilic group.
<3> The oil-in-water emulsion cosmetic according to <1> or <2>, wherein the self-water-dispersible crosslinked silicone elastomer is a (dimethicone/(PEG-10/15)) crosspolymer.
<4> The oil-in-water emulsion cosmetic according to any one of <1> to <3>, wherein the water-soluble polymer is an acrylic acid·alkyl methacrylate copolymer.
<5> The oil-in-water emulsion cosmetic according to any one of <1> to <4>, containing both the nonionic surfactant and the water-soluble polymer, wherein the nonionic surfactant content is 0.3 mass% or less.
<6> The oil-in-water emulsion cosmetic according to any one of <1> to <5>, wherein the silicone oil content is 10 to 40 mass%.
<7> The oil-in-water emulsion cosmetic according to any one of <1> to <6>, further containing a water-based thickener.
<8> The oil-in-water emulsion cosmetic according to <7>, wherein the water-based thickener contains a carboxyvinyl polymer.
<9> The oil-in-water emulsion cosmetic according to any one of <1> to <8>, further containing a silicone-based surfactant having HLB of 6 or less as an auxiliary emulsifying agent.
<10> The oil-in-water emulsion cosmetic according to <9>, wherein the silicone-based surfactant content is 0.01 to 0.3 mass%.

### ADVANTAGEOUS EFFECTS OF INVENTION

According to the invention, an oil-in-water emulsion cosmetic having a low viscosity and excellent storage stability can be obtained. Therefore, a cosmetic which has further improved feeling upon use and achieves excellent smoothness/evenness of makeup application as well as having the conventional excellent feeling upon use and the uneven-texture correcting effect can be provided.

### DESCRIPTION OF EMBODIMENTS

### «Oil-In-Water Emulsion Cosmetic»

The oil-in-water emulsion cosmetic (sometimes simply referred to as a "cosmetic" below) according to the invention contains 2 to 10 mass% of a self-water-dispersible crosslinked silicone elastomer (sometimes simply referred to as a "silicone elastomer" below), at least one of a nonionic surfactant having HLB of 12.5 or more and a water-soluble polymer having emulsifying capability and a silicone oil having a kinematic viscosity at 25°C of 20 mm²/s or less and is characterized by having a kinematic viscosity at 25°C of 10000 mPa·s or less.

### <Self-Water-Dispersible Crosslinked Silicone Elastomer>

The self-water-dispersible crosslinked silicone elastomer (also referred to as a "silicone elastomer" below) of the invention has sufficient hydrophilic groups and is able to disperse in water as the elastomer alone without the existence of a dispersing agent or the like (self-dispersing in water), and the molecule can be appropriately designed.

The kinematic viscosity at 25°C of a cosmetic using the conventional crosslinked silicone elastomer has generally been 20000 mPa·s or more in view of the storage stability. However, when the silicone elastomer of the invention is used, sufficient dispersibility and storage stability (long-term dispersibility) can be achieved even with a kinematic viscosity at 25°C of 10000 mPa·s or less.

The viscosity of the cosmetic is preferably 10000 mPa·s or less in view of the handling property or the feeling upon use. On the other hand, an extremely low viscosity is not preferable for excellent use feeling or adhesion of the cosmetic, and the viscosity is preferably 2000 mPa·s or more, more preferably 3000 mPa·s or more.

The silicone elastomer is generally used in a state of being kneaded in a silicone oil, and its concentration is generally around 10 to 40 mass%. The dispersibility of the silicone elastomer of the invention in water is evaluated by the following method.

A silicone elastomer paste in an amount of 3 g in a state of being kneaded in a silicone oil with appropriate dispersibility is added to 30 g of ion-exchanged water, and after shaking/stirring for a minute, the mixture is visually observed also after three minutes. The dispersibility is evaluated by this method. When no agglomerate suspended matter is observed in the upper part and when milkiness of the water part and clear dispersion of the silicone oil and the silicone elastomer are confirmed by the method, the silicone elastomer is determined to have self-water-dispersibility. It is essential in the invention to use a silicone elastomer having such self-water-dispersibility.

An example of the silicone elastomer is a copolymer of a silicone compound and a hydrophilic monomer. Examples of the hydrophilic group are a polyether group and a glyceryl group, but the hydrophilic group is not limited to the examples.

The hydrophilic group in the silicone elastomer is not particularly restricted as long as the hydrophilic group is generally used for producing an amphipathic silicone compound, and examples include polyethylene glycol (PEG), polypropylene glycol (PPG) polyglycerol and the like. Of these examples, polyethylene glycol is preferably contained. Only a kind of hydrophilic group may be contained, or more than one kind thereof may be contained.

An example of the copolymer of a silicone compound and a hydrophilic monomer is a crosslinked organopolysiloxane obtained by reacting an organohydrogen polysiloxane represented by general formula (I) below and a polyoxyalkylene compound represented by general formula (II) below in the presence of a catalyst for hydrosilylation reaction.

R¹ₐR²_{b}SiO_{(4-a-b)/2} (I)

(In the formula, R¹'s may be the same or different from each other and are substituted or unsubstituted monovalent hydrocarbon groups having 1 to 30 carbon atoms and having no alkenyl group. R²'s may be the same or different from each other and are hydrogen atoms or alkyl groups having 1 to 30 carbon atoms. a and b are positive numbers which satisfy 1.0≤a≤2.5, 0.001≤b≤1.2 and 1.0≤a+b≤2.6.)

C_{c}H_{2c-1}O(C₂H₄O)_{d}(C₃H₆O)ₑC_{c}H_{2c-1} II

(In the formula, c is an integer of 2 to 6, and d and e are integers which satisfy 5≤d≤200 and 0≤e≤200.)

In order that the crosslinked organopolysiloxane has sufficient hydrophilicity, the proportion of the polyoxyethylene unit (C₂H₄O) in the entire crosslinked organopolysiloxane is preferably 20 to 50 wt%, more preferably 30 to 50wt%.

Examples of R¹ in general formula (I) include alkyl groups such as methyl group, ethyl group, propyl group, butyl group, pentyl group, hexyl group, heptyl group, octyl group, nonyl group and decyl group; saturated alicyclic hydrocarbon groups such as cyclopentyl group and cyclohexyl group; aryl groups such as phenyl group and tolyl group; fluorine-substituted alkyl groups such as trifluoropropyl group, nonafluorohexyl group and heptadecylfluorodecyl group and the like. The alkyl groups in R² in general formula (I) are methyl group, ethyl group, propyl group, butyl group, pentyl group, hexyl group, heptyl group, octyl group, nonyl group, decyl group or the like.

The organohydrogen polysiloxane may have any of linear, branched and cyclic structures but preferably has a linear structure for smooth progress of the polymerization reaction.

In general formula (II), d is more preferably 5≤d≤100. e is more preferably 5≤e≤100.

More specifically, the silicone elastomer is more preferably a (dimethicone/(PEG-10/15)) crosspolymer.

The silicone elastomer has sufficient hydrophilic groups and thus can disperse in water without the use of a dispersing agent. Accordingly, it is not always necessary to add a dispersing agent to the cosmetic, and even when a dispersing agent is added, a smaller amount than the conventional amount is sufficient. Thus, an oil-in-water emulsion cosmetic which has a low viscosity but has excellent dispersibility and storage stability (long-term dispersibility) can be obtained.

By adding the silicone elastomer to the cosmetic, excellent feeling upon use and an uneven-texture correcting effect are obtained. Here, in the invention, because the silicone elastomer can be added in a larger amount than the conventional amount as a result of not adding a dispersing agent or adding a small amount, it becomes possible not only to provide the excellent feeling upon use and the uneven-texture correcting effect but also to further improve the feeling upon use and achieve extremely excellent smoothness/evenness of makeup application.

Moreover, because the silicone elastomer of the invention is used, the entire cosmetic is not sticky, and the above effects can be obtained, even when a high amount of a moisturizing agent which promotes stickiness such as glycerol is added.

By controlling the silicone elastomer content of the cosmetic to 2 mass% or more, excellent feeling upon use, an uneven-texture correcting effect and smoothness/evenness of makeup application can be achieved. In particular, when the cosmetic is a foundation, clogging can be prevented, and the cosmetic can be applied well on skin. The silicone elastomer content is more preferably 3 mass% or more.

On the other hand, the cosmetic becomes considerably heavy upon use when the silicone elastomer is contained excessively, and thus the amount is 10 mass% or less.

### <Nonionic Surfactant, Water-Soluble Polymer>

The nonionic surfactant having HLB of 12.5 or more and the water-soluble polymer having emulsifying capability are both components which improve the dispersibility or the emulsifying property and also contribute to better dispersibility of the silicone elastomer. The HLB of the nonionic surfactant is more preferably 14 or more.

The HLB indicates the degree of affinity to water and an oil, and the cosmetic becomes an oil-in-water type by adding the nonionic surfactant having HLB of 12.5 or more. The nonionic surfactant is not particularly limited as long as the nonionic surfactant has HLB of 12.5 or more and is generally used for cosmetics, and examples include glycerol or polyglycerol fatty acid esters, propylene glycol fatty acid esters, polyoxyethylene (POE) sorbitan fatty acid esters, POE sorbitol fatty acid esters, POE glycerol fatty acid esters, POE fatty acid esters, POE alkylethers, POE alkylphenyl ethers, POE·polyoxypropylene (POP) alkylethers, POE castor oil, POE hydrogenated castor oil, POE beeswax·lanolin derivatives, alkanolamides, POE propylene glycol fatty acid esters, POE alkylamines, POE fatty acid amides, POE-modified silicone, POE·POP-modified silicone and the like. Of these examples, POE castor oil or POE hydrogenated castor oil is particularly preferable. A kind thereof may be used, or more than one kind thereof may be used. When more than one kind of nonionic surfactant are used, the weighted average of the HLB values of the nonionic surfactants is the HLB value of the entire nonionic surfactant.

The water-soluble polymer having emulsifying capability is not particularly limited, either, as long as the water-soluble polymer is generally used for cosmetics, and examples include an acrylic acid·alkyl methacrylate copolymer. Specific examples include Pemulen (registered trademark) TR-1 and Pemulen (registered trademark) TR-2 manufactured by Lubrizol (both are (acrylate/alkyl acrylate (C10-30)) cross polymers) and the like.

The cosmetic according to the invention contains at least one of the nonionic surfactant having HLB of 12.5 or more and the water-soluble polymer having emulsifying capability and more preferably contains both the nonionic surfactant and the water-soluble polymer.

The nonionic surfactant content is preferably 0.3 mass% or less in view of the use feeling and is more preferably 0.2 mass% or less.

In view of securing both stability and a viscosity range (2000 to 10000 mPa·s) for excellent use feeling, the total amount of the nonionic surfactant and the water-soluble polymer is preferably 0.2 mass% or more, more preferably 0.25 mass% or more and is preferably 1 mass% or less, more preferably 0.5 mass% or less.

### <Silicone Oil>

The cosmetic according to the invention preferably contains an oil content in addition to the silicone elastomer in view of protecting the skin on which the cosmetic is applied and more preferably contains a silicone oil because the silicone oil has a composition similar to that of the silicone elastomer, further improves the emulsifying property and contributes to better dispersibility of the silicone elastomer. As the silicone oil, a silicone oil having a kinematic viscosity at 25°C of 20 mm²/s or less is further preferable because a cosmetic which is less sticky can be provided.

The silicone oil content is preferably 10 mass% or more, more preferably 15 mass% or more in view of obtaining the above effects. Excessive addition decreases the stability and causes oily use feeling, and thus the amount is preferably 40 mass% or less, more preferably 30 mass% or less.

The silicone oil may be volatile or nonvolatile and is not particularly limited as long as the silicone oil is generally used for cosmetics. Examples include linear or cyclic polysiloxanes such as methylpolysiloxane, diphenylsiloxy phenyl trimethicone, dimethylpolysiloxane, methylphenylpolysiloxane, methylhydrogen polysiloxane, decamethylpolysiloxane, dodecamethylpolysiloxane, tetramethyltetrahydrogen polysiloxane, cyclotetradimethylsiloxane and cyclopentadimethylsiloxane. A kind thereof may be used, or more than one kind thereof may be used.

An oil content other than the silicone oil may also be contained, and examples include oils and fats, waxes, hydrocarbons, higher fatty acids, higher alcohols, synthetic ester oils, natural ester oils and the like. For the purpose of preventing sunburn, a polar oil (an oil-soluble ultraviolet absorber) as an ultraviolet absorber is also preferably used.

Examples of the oil-soluble ultraviolet absorber include cinnamic acid-based ultraviolet absorbers such as octyl cinnamate, ethyl-4-isopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octylmethoxycinnamate, 2-ethoxyethyl-p-methoxycinnamate, cyclohexyl-p-methoxycinnamate, ethyl-α-cyano-β-phenyl cinnamate and 2-ethylhexyl-α-cyano-β-phenyl cinnamate.

The total amount of the silicone oil and the oil-soluble ultraviolet absorber is preferably 40 mass% or less, more preferably 30 mass% or less and is preferably 10 mass% or more, more preferably 15 mass% or more.

When the other oil content is contained, the total amount of the silicone oil, the oil-soluble ultraviolet absorber and the other oil content is preferably in the above range.

### <Water-Based Thickener>

In view of controlling the viscosity to a certain value or more and improving the stability, the cosmetic preferably further contains a water-based thickener, and the viscosity of the cosmetic can be controlled to preferably 2000 mPa·s or more, more preferably 3000 mPa·s or more.

The water-based thickener is not particularly limited as long as the water-based thickener is generally used for cosmetics, and for example, a carboxyvinyl polymer is preferable in view of the refreshing use feeling. In addition, examples include the water-based thickeners shown below. A kind thereof may be used, or more than one kind thereof may be used.

Water-soluble polymers such as guar gum, xanthan gum and polyvinyl alcohol; taurate-based polymer thickeners such as polymers and/or copolymers (including crosslinked polymers) having 2-acrylamido-2-propane sulfonic acid (acryloyldimethyltauric acid) or a salt thereof (AMPS structure) as a structural unit; acrylate-based synthetic polymer thickeners; other thickeners and the like can be used.

As the taurate-based polymer thickeners, for example, an (ammonium acryloyldimethyltaurate/beheneth-25 methacrylate) crosspolymer (Aristoflex (registered trademark) HMB, Clariant Japan KK), an (ammonium acryloyldimethyltaurate/vinylpyrrolidone) copolymer (Aristoflex (registered trademark) AVC, Clariant Japan KK), an (ammonium acryloyldimethyltaurate/carboxylethyl acrylate) crosspolymer (Aristoflex (registered trademark) TAC, Clariant Japan KK), a polyacrylate crosspolymer-11 (Aristoflex (registered trademark) Velvet, Clariant Japan KK), a (dimethylacrylamide/sodium acryloyldimethyltaurate) crosspolymer, a (hydroxyethyl acrylate/sodium acryloyldimethyltaurate) copolymer (SEPINOV EMT10 PINOV, SEPPIC), a (sodium acrylate/acryloyldimethyltaurate/dimethyl acrylamide) crosspolymer (SEPINOV P88, SEPPIC), a (sodium acrylate/sodium acryloyldimethyltaurate) copolymer (SIMULGEL EG, SEPPIC), a (sodium acryloyldimethyltaurate/lauryl methacrylamide) copolymer (AMO-51, Daito Kasei Kogyo Co., Ltd.), an (acrylamide/sodium acryloyldimethyltaurate/acrylic acid) copolymer (Acudyne SCP, Dow Chemical Company) and the like can be used.

As the acrylate-based synthetic polymer thickeners, for example, an (acrylate/steareth-20 methacrylate) copolymer (ACULYN (registered trademark) 22, Dow Chemical Company), an (acrylate/C10-30 alkyl acrylate) crosspolymer (PEMULEN (registered trademark) TR-1 or PEMULEN (registered trademark) TR-2, Lubrizol) and the like can be used.

As the other thickeners, for example, gum arabic, carrageenan, gum karaya tragacanth gum, carob gum, quince seed (marmelo), casein, dextrin, gelatin, sodium pectate, sodium alginate, methylcellulose, ethylcellulose, carboxymethyl cellulose (CMC), hydroxyethyl cellulose, hydroxypropyl cellulose, polyvinyl alcohol (PVA), polyvinyl methyl ether (PVM), PVP (polyvinylpyrrolidone), sodium polyacrylate, carboxyvinyl polymer, locust bean gum, guar gum, tamarind gum, cellulose dialkyl dimethyl ammonium sulfate, xanthan gum, magnesium aluminum silicate, bentonite, hectorite, magnesium aluminum silicate (Veegum), Laponite, silicic anhydride and the like can be used.

A preferable water-based thickener content of the cosmetic differs with the kind of the water-based thickener used. When a carboxyvinyl polymer is used as the water-based thickener, for example, the amount is preferably 0.05 to 0.3 mass%.

### <Auxiliary Emulsifying Agent>

The cosmetic according to the invention preferably further contains an auxiliary emulsifying agent for further improving the emulsifying property.

As the auxiliary emulsifying agent, an amphipathic component which is compatible to the silicone oil and which has HLB of 6 or less is preferable, and a silicone-based surfactant having HLB of 6 or less is more preferable. The silicone-based surfactant having HLB of 6 or less is polyether-modified silicone, alkyl·polyether-modified silicone, polyglycerol-modified silicone, alkyl·polyglycerol-modified silicone or the like. A kind of the auxiliary emulsifying agents may be used, or more than one kind thereof may be used.

When a silicone-based surfactant having HLB of 6 or less is used as the auxiliary emulsifying agent, it is preferable to control the amount to 0.01 mass% or more because the emulsifying property can be further improved, and the amount is more preferably 0.03 mass% or more. On the other hand, the cosmetic may become a water-in-oil emulsion cosmetic with excessive addition, and thus the amount is preferably 0.5 mass% or less, more preferably 0.3 mass% or less.

### <Other Components>

The cosmetic can contain any other components in addition to the components described above in the range in which the effects of the invention are not impaired.

Examples of the other components include moisturizing agents, pH controllers, neutralizing agents, antioxidants, alcohols, preservatives, antibacterial agents, medicines, (plant) extracts, fragrances, dyes, ultraviolet absorbers other than the oil-soluble ultraviolet absorbers described above and the like, and the conventionally generally used kinds thereof can be used in general amounts.

The moisturizing agents are polyhydric alcohols such as glycerol, diethylene glycol, butylene glycol, dipropylene glycol and polyethylene glycol, amino acids, nucleic acids, proteins such as collagen and elastin, mucopolysaccharides such as hyaluronic acid and chondroitin sulfate and the like.

The pH controllers are lactic acid, citric acid, glycolic acid, succinic acid, tartaric acid, dl-malic acid, potassium carbonate, sodium hydrogen carbonate, ammonium hydrogen carbonate and the like.

The antioxidants are ascorbic acid, α-tocopherol, dibutylhydroxytoluene, butylhydroxyanisole and the like.

As the alcohols, ethyl alcohol can be used.

The preservatives and the antibacterial agents include paraoxy benzoate ester, phenoxyethanol, benzoic acid, salicylic acid, carbolic acid, sorbic acid, parachlormetacresol, hexachlorophene, benzalkonium chloride, chlorhexidine chloride, trichlorocarbanilide, photosensitizers and the like.

### «Production Method»

The oil-in-water emulsion cosmetic according to the invention can be produced by the following method, for example. A powder dispersion liquid is obtained in advance by mixing and dispersing the oil phase components (including hydrophobic powder and the crosslinked silicone elastomer) of the components with a homomixer, and the water phase components are mixed and emulsified with a homomixer while the obtained powder dispersion liquid is added to the water phase components. When powder particles which have larger particle sizes than those of the generated emulsified particles exist at this point, a part of the powders escapes from the oil phase and forms agglomerates through the homomixer treatment, and thus the average particle size of the powder is preferably smaller than the emulsified particle size. By a method of making the stirring conditions severe or taking a long time with respect to the conditions for obtaining the dispersion liquid or another method, the particle size of the dispersed powder can be made sufficiently small, and pulverized powder which is sufficiently smaller than the emulsified particle size can be obtained.

In the range in which the effects of the invention are not impaired, components which are generally used for cosmetics such as the moisturizing agents, the ultraviolet absorbers, the pH controllers, the neutralizing agents, the antioxidants, the alcohols, the preservatives, the antibacterial agents, the medicines, the extracts, the fragrances and the dyes described above can be added to the oil phase components or the water phase components.

### «Applications»

The product form of the oil-in-water emulsion cosmetic according to the invention is any form, and for example, the oil-in-water emulsion cosmetic is preferably used for facial cosmetics such as face lotions, milky lotions and creams; makeup cosmetics such as makeup bases, foundations, lipsticks, eyeshadows, blushers, eyeliners, mascaras and sunscreens; body cosmetics; fragrance cosmetics; and the like. Of these examples, a cosmetic which is used before the base makeup products such as makeup bases or milky lotions is more preferable in view of improving the smoothness/evenness of makeup application of the base makeups such as foundations.

### EXAMPLES

The invention is specifically explained below referring to test examples, but the invention is not limited to the examples.

In this regard, "%" which indicates the amount of a component means "mass%".

### «Examples 1 to 8 and Comparative Examples 1 to 5»

### <Evaluation Method>

The viscosities, the storage stability and the feeling upon use of obtained oil-in-water emulsion cosmetics and the smoothness/evenness of makeup application of a solid powder foundation were evaluated.

The evaluation methods are as described below, and the results are shown in Tables 2 and 3.

### (Kinematic Viscosity)

Regarding the viscosities of the oil-in-water emulsion cosmetics, the kinematic viscosities (mPa·s) at 25°C were measured using Digital Vismetron VDA2 manufactured by Shibaura system with a rotor rotation speed of 12 rpm.

The criteria for evaluation are as follows.
B: The kinematic viscosity is 10000 mPa·s or less.
D: The kinematic viscosity exceeds 10000 mPa·s.

### (Storage Stability)

After the production, the oil-in-water emulsion cosmetics were left still at 50°C for a month, and the changes in appearances were observed visually. The criteria for evaluation are as follows.
A: Separation of the oil content or the elastomer component is not observed.
B: Slight separation of the oil content or the elastomer component is observed on the surface of the emulsion.
D: Considerable separation of the oil content or the elastomer component is observed.

### (Feeling Upon Use)

Five analysts specialized in cosmetics applied the oil-in-water emulsion cosmetics on the face and gave scores based on the stickiness and the sliminess (1 point for bad, 2 points for slightly bad, 3 points for normal, 4 points for slightly good, and 5 points for good), and the evaluation was made using the average scores. The criteria for evaluation are as follows.
A: The average score is 4.5 or more.
B: The average score is 3.5 or more and less than 4.5.
C: The average score is 2.5 or more and less than 3.5.
D: The average score is 1.0 or more and less than 2.5 or less.

### (Smoothness/evenness of makeup application)

A solid powder foundation was produced with the prescription and by the production method shown in Table 1 below. Analysts specialized in cosmetics applied an oil-in-water emulsion cosmetic on the face and then applied the solid powder foundation obtained above on the face. The analysts gave the smoothness/evenness of makeup application of the solid powder foundation scores (1 point for bad, 2 points for slightly bad, 3 points for normal, 4 points for slightly good, and 5 points for good), and the evaluation was made using the average scores. The criteria for evaluation are as follows. The smoothness/evenness of makeup application here means the prevention of clogging and good·even adhesion onto the skin.
A: The average score is 4.5 or more.
B: The average score is 3.5 or more and less than 4.5.
C: The average score is 2.5 or more and less than 3.5.
D: The average score is 1.0 or more and less than 2.5.

[Table 1]

**Table 1; Prescription Example (mass%)**

| | | |
|---|---|---|
| Powder part | Silicone-treated talc | 21.7 |
| | Sericite | 20 |
| | Synthetic Fluorphlogopite | 20 |
| | Boron nitride | 3 |
| | Spherical PMMA powder | 5 |
| | Spherical silica powder | 5 |
| | Spherical nylon powder | 3 |
| | Silicone-treated titanium oxide | 10 |
| | Silicone-treated iron oxide (red) | 0.5 |
| | Silicone-treated iron oxide (yellow) | 1.5 |
| | Silicone-treated iron oxide (black) | 0.1 |
| | Ethylparaben | 0.2 |
| Oil phase part | Dimethicone (6 mm²/s) | 4.5 |
| | Vaseline | 0.5 |
| | Trioctanoin | 3 |
| | Diisostearyl malate | 1 |
| | Sorbitan sesquiisostearate | 1 |

### • Production Method

After stirring and mixing the powder part of the prescription example above, the oil phase part of the prescription example which had been evenly heated and solubilized was added and mixed, and the mixture was pulverized with a pulverizer. The obtained powders were packed in an inner plate and formed by applying pressure, and a solid powder foundation was thus obtained.

### <Production of Oil-In-Water Emulsion Cosmetics>

The water phase components shown in Tables 2 and 3 except for ethanol were heated and solubilized and then cooled to normal temperature, and ethanol was then added. The oil phase components which had been mixed and dispersed in advance with a homomixer were added, and the mixture was emulsified with a homogenizer. The oil-in-water emulsion cosmetics were thus produced.

In Tables 2 and 3, the (dimethicone/(PEG-10/15)) crosspolymer corresponds to the self-water-dispersible crosslinked silicone elastomer. Polyoxyethylene hydrogenated castor oil (60 E.O.) (HLB=14) (=Nikkol HCO-60 (manufactured by Nikko Chemicals Co., Ltd.)) and polyoxyethylene hydrogenated castor oil (20 E.O.) (HLB=10.5) (=Nikkol HCO-20 (manufactured by Nikko Chemicals Co., Ltd.)) correspond to the nonionic surfactants. The acrylic acid·alkyl methacrylate copolymer (=Pemulen (registered trademark) TR-2 (Lubrizol)) corresponds to the water-soluble polymer having emulsifying capability. Diphenylsiloxy phenyl trimethicone and methylpolysiloxane (6cs) correspond to the silicone oils having a kinematic viscosity at 25°C of 20 mm²/s or less, and the carboxyvinyl polymer corresponds to the water-based thickener. PEG-10 dimethicone (=KF-6017 (Shin-Etsu Chemical Co., Ltd.)) corresponds to the silicone-based surfactant having HLB of 6 or less. The HLB of the nonionic surfactants of Comparative Example 5, which contained polyoxyethylene hydrogenated castor oil (60 E.O.) and polyoxyethylene hydrogenated castor oil (20 E.O.) each at a concentration of 0.15 mass%, is 12.25 from the weighted average.

[Table 2]

**Table 2 (mass%)**

| Test Example | | Example 1 | Example 2 | Example 3 | Example 4 | Example 5 | Example 6 | Example 7 | Example 8 |
|---|---|---|---|---|---|---|---|---|---|
| Water phase component | Carboxyvinyl polymer | 0.15 | 0.15 | 0.15 | 0.15 | 0.15 | 0.15 | 0.15 | 0.15 |
| | Acrylic acid·alkyl methacrylate copolymer | 0.08 | | 0.04 | 0.08 | 0.08 | 0.08 | 0.08 | 0.08 |
| | Ethanol | 3 | 3 | 3 | 3 | 3 | 3 | 3 | 3 |
| | Polyoxyethylene hydrogenated castor oil (60 E.O.) (HLB14) | 0.15 | 0.15 | 0.15 | 0.15 | 0.15 | 0.15 | 0.15 | 0.15 |
| | Polyoxyethylene hydrogenated castor oil (20 E.O.) (HLB10.5) | | | | | | | | |
| | Dipropylene glycol | 5 | 5 | 5 | 5 | 5 | 5 | 5 | 5 |
| | 1,3 Butylene glycol | 4 | 4 | 4 | 4 | 4 | 4 | 4 | 4 |
| | Glycerin | 3 | 3 | 3 | 3 | 3 | 3 | 3 | 3 |
| | Phenoxyethanol | 0.5 | 0.5 | 0.5 | 0.5 | 0.5 | 0.5 | 0.5 | 0.5 |
| | Ion-exchanged water | Balance | Balance | Balance | Balance | Balance | Balance | Balance | Balance |
| Oil phase component | (Dimethicone/(PEG-10/15)) crosspolymer | 3.5 | 3.5 | 3.5 | 3.5 | 7 | 2 | 7 | 3.5 |
| | (Dimethicone/phenylvinyldimethicone) crosspolymer | | | | | | | | |
| | Diphenylsiloxy phenyl trimethicone | 6.5 | 6.5 | 6.5 | 6.5 | 13 | 3 | 13 | 6.5 |
| | PEG-10 dimethicone | | | | 0.1 | 0.1 | 0.1 | 0.05 | |
| | Methylpolysiloxane (6cs) | 6 | 6 | 6 | 6 | 6 | 6 | 6 | |
| | Methylpolysiloxane (100cs) | | | | | | | | 6 |
| | Glyceryl tri(2-ethylhexanoate) | 1.5 | 1.5 | 1.5 | 1.5 | 1.5 | 1.5 | 1.5 | 1.5 |
| | Diisostearyl malate | 1 | 1 | 1 | 1 | 1 | 1 | 1 | 1 |
| Evaluation Results | Kinetic viscosity at 25°C (mPa·s) | B | B | B | B | B | B | B | B |
| | Storage stability | B | B | B | A | A | A | A | B |
| | Feeling upon use (reduced stickiness or sliminess) | B | B | B | B | A | B | A | C |
| | Smoothness/evenness of makeup application | B | B | B | B | A | C | A | C |

[Table 3]

**Table 3 (mass%)**

| Test Example | | Comparative Example 1 | Comparative Example 2 | ComparativeExample 3 | ComparativeExample 4 | ComparativeExample 5 |
|---|---|---|---|---|---|---|
| | Carboxyvinyl polymer | 0.15 | 0.4 | 0.15 | 0.15 | 0.15 |
| | Acrylic acid·alkyl methacrylate copolymer | 0.08 | 0.08 | 0.08 | 0.08 | 0.08 |
| | Ethanol | 3 | 3 | 3 | 3 | 3 |
| | Polyoxyethylene hydrogenated castor oil (60 E.O.) (HLB14) | 0.15 | 0.15 | 0.15 | 0.15 | 0.15 |
| Water phase | Polyoxyethylene hydrogenated castor oil (20 E.O.) (HLB10.5) | | | | 0.15 | |
| component | Dipropylene glycol | 5 | 5 | 5 | 5 | 5 |
| | 1,3 Butylene glycol | 4 | 4 | 4 | 4 | 4 |
| | Glycerin | 3 | 3 | 3 | 3 | 3 |
| | Phenoxyethanol | 0.5 | 0.5 | 0.5 | 0.5 | 0.5 |
| | Ion-exchanged water | Balance | Balance | Balanche | Balanche | Balanche |
| | (Dimethicone/(PEG-10/15)) crosspolymer | | 3.5 | 10.5 | 3.5 | 0.35 |
| | (Dimethicone/phenylvinyldimethicone) crosspolymer | 2 | | | | |
| | Diphenylsiloxy phenyl trimethicone | 8 | 6.5 | 19.5 | 6.5 | 0.65 |
| Oil phase | PEG-10 dimethicone | | | 0.1 | | 0.1 |
| component | Methylpolysiloxane (6cs) | 6 | 6 | 6 | 6 | 6 |
| | Methylpolysiloxane (100cs) | | | | | |
| | Glyceryl tri(2-ethylhexanoate) | 1.5 | 1.5 | 1.5 | 1.5 | 1.5 |
| | Diisostearyl malate | 1 | 1 | 1 | 1 | 1 |
| | Kinetic viscosity at 25°C (mPa·s) | B | D | D | B | B |
| Evaluation | Storage stability | D | A | D | D | A |
| Results | Feeling upon use (reduced stickiness or sliminess) | B | D | D | B | A |
| | Smoothness/evenness of makeup application | B | B | B | B | D |

### «Examples 9 and 10»

The water phase components shown in Table 4 or 5 except for ethanol were heated and solubilized and then cooled to normal temperature, and ethanol was then added. The oil phase components which had been mixed and dispersed in advance with a homomixer were added, and the mixture was emulsified with a homogenizer. Then, the powder part was added, and the mixture was dispersed again with a homogenizer. The oil-in-water emulsion cosmetics of the invention were thus produced.

In Tables 4 and 5, the (dimethicone/(PEG-10/15)) crosspolymer corresponds to the self-water-dispersible crosslinked silicone elastomer. Polyoxyethylene hydrogenated castor oil (100 E.O.) (HLB=16.5) (=Nikkol HCO-100 (manufactured by Nikko Chemicals Co., Ltd.)) and polyoxyethylene hydrogenated castor oil (60 E.O.) (HLB=14) (=Nikkol HCO-60 (manufactured by Nikko Chemicals Co., Ltd.)) correspond to the nonionic surfactants. The acrylic acid·alkyl methacrylate copolymer (=Pemulen (registered trademark) TR-2 (Lubrizol)) corresponds to the water-soluble polymer having emulsifying capability. Methylpolysiloxane (6cs) and diphenylsiloxy phenyl trimethicone correspond to the silicone oils having a kinematic viscosity at 25°C of 20 mm²/s or less, and the carboxyvinyl polymer corresponds to the water-based thickener. PEG-10 dimethicone (=KF-6017 (Shin-Etsu Chemical Co., Ltd.)) corresponds to the silicone-based surfactant having HLB of 6 or less. Lauryl PEG-9 polydimethylsiloxyethyl dimethicone used was KF6038 manufactured by Shin-Etsu Chemical Co., Ltd.

[Table 4]

**Table 4 (mass%)**

| Test Example | | Example 9 |
|---|---|---|
| Water phase component | Carboxyvinyl polymer | 0.15 |
| | Acrylic acid·alkyl methacrylate copolymer | 0.08 |
| | Ethanol | 3 |
| | Polyoxyethylene hydrogenated castor oil (100 E.O.) (HLB16.5) | 0.2 |
| | Dipropylene glycol | 5 |
| | 1,3 Butylene glycol | 4 |
| | Glycerin | 3 |
| | Phenoxyethanol | 0.5 |
| | Ion-exchanged water | Balance |
| Oil phase component | (Dimethicone/(PEG-10/15)) crosspolymer | 3.5 |
| | Diphenylsiloxy phenyl trimethicone | 6.5 |
| | PEG-10 dimethicone | 0.05 |
| | Methylpolysiloxane (6cs) | 6 |
| | Octylmethoxycinnamate | 3 |
| Powder part | Iron oxide red | 0.001 |
| | Iron oxide yellow | 0.002 |
| | Mica titanium | 0.5 |

[Table 5]

**Table 5 (mass%)**

| Test Example | | Example 10 |
|---|---|---|
| Water phase component | Carboxyvinyl polymer | 0.15 |
| | Acrylic acid·alkyl methacrylate copolymer | 0.08 |
| | Ethanol | 3 |
| | Polyoxyethylene hydrogenated castor oil (60 E.O.) (HLB14) | 0.2 |
| | Dipropylene glycol | 3 |
| | 1,3 Butylene glycol | 2 |
| | Glycerin | 10 |
| | Phenoxyethanol | 0.5 |
| | Ion-exchanged water | Balance |
| Oil phase component | (Dimethicone/(PEG-10/15)) crosspolymer | 3.5 |
| | Diphenylsiloxy phenyl trimethicone | 6.5 |
| | Lauryl PEG-9 polydimethylsiloxyethyl dimethicone | 0.05 |
| | Methylpolysiloxane (6cs) | 6 |
| | Octylmethoxycinnamate | 5 |
| Powder part | Hydrophilic spherical (methyl methacrylate/methacrylic acid (PEG-4/PEG-3)) crosspolymer | 5 |
| | Iron oxide yellow | 0.002 |
| | Iron oxide red | 0.001 |

Although the invention has been explained in detail referring to specific embodiments, it is obvious to one skilled in the art that various changes and modifications can be made without departing from the spirit and the scope of the invention. The present application is based on a Japanese patent application filed on August 1, 2018 (patent application No. 2018-145176), and the contents thereof are incorporated here by reference.

## Claims

1. An oil-in-water emulsion cosmetic, comprising:
2 to 10 mass% of a self-water-dispersible crosslinked silicone elastomer,
at least one of a nonionic surfactant having HLB of 12.5 or more and a water-soluble polymer having emulsifying capability and
a silicone oil having a kinematic viscosity at 25°C of 20 mm²/s or less, wherein the oil-in-water emulsion cosmetic has a kinematic viscosity at 25°C of 10000 mPa·s or less.

2. The oil-in-water emulsion cosmetic according to claim 1,
wherein the self-water-dispersible crosslinked silicone elastomer comprises polyethylene glycol as a hydrophilic group.

3. The oil-in-water emulsion cosmetic according to claim 1 or 2,
wherein the self-water-dispersible crosslinked silicone elastomer is a (dimethicone/(PEG-10/15)) crosspolymer.

4. The oil-in-water emulsion cosmetic according to any one of claims 1 to 3,
wherein the water-soluble polymer is an acrylic acid·alkyl methacrylate copolymer.

5. The oil-in-water emulsion cosmetic according to any one of claims 1 to 4, comprising:
both the nonionic surfactant and the water-soluble polymer,
wherein the nonionic surfactant content is 0.3 mass% or less.

6. The oil-in-water emulsion cosmetic according to any one of claims 1 to 5,
wherein the silicone oil content is 10 to 40 mass%.

7. The oil-in-water emulsion cosmetic according to any one of claims 1 to 6, further comprising:
a water-based thickener.

8. The oil-in-water emulsion cosmetic according to claim 7,
wherein the water-based thickener comprises a carboxyvinyl polymer.

9. The oil-in-water emulsion cosmetic according to any one of claims 1 to 8, further comprising:
a silicone-based surfactant having HLB of 6 or less as an auxiliary emulsifying agent.

10. The oil-in-water emulsion cosmetic according to claim 9,
wherein the silicone-based surfactant content is 0.01 to 0.3 mass%.
